# FASCICULE DE BREVET EUROPEEN

(11) **EP 0 491 014 B1**
(45) Date de publication et mention de la délivrance du brevet: **04.09.1996**
(21) Numéro de dépôt: 91911095.7
(22) Date de dépôt: 06.06.1991
(51) Int. Cl.: C07K 7/00, G01N 33/564, A61K 39/395, C12P 21/08

(54) **PEPTIDES DE L'ANTIGENE Sm-D ET LEUR UTILISATION NOTAMMENT POUR LE DIAGNOSTIC DU LUPUS ERYTHEMATEUX DISSEMINE**
PEPTIDE VON SM-D-ANTIGEN, DEREN BENÜTZUNG ZUM NACHWEIS VON DISSEMINIERTEN LUPUS-ERYTHEMATEUX
Sm-D ANTIGEN PEPTIDES AND THEIR USE, IN PARTICULAR, FOR THE DIAGNOSIS OF SYSTEMIC LUPUS ERYTHEMATOSUS

(30) Priorité: 06.06.1990 FR 9007029
(43) Date de publication de la demande: 24.06.1992
(73) Titulaire: PASTEUR SANOFI DIAGNOSTICS, F-92430 Marnes La Coquette (FR)
(72) Inventeur: VAN REGENMORTEL, Marc H.V., F-67000 Strasbourg (FR); MULLER, Sylviane, F-67000 Strasbourg (FR); BRIAND, Jean-Paul, F-67000 Strasbourg (FR); BARAKAT, Samira, F-67084 Strasbourg Cedex (FR); WEBER, Jean-Christophe, F-67000 Strasbourg (FR)
(74) Mandataire: Polus, Camille
(86) Numéro de dépôt international: FR9100445
(87) Numéro de publication internationale: WO9118920

(56) Documents cités:
- EP-A- 0 295 719
- WO-A-86/01210

## Description

La présente invention a pour objet des peptides susceptibles d'être reconnus par les anticorps présents dans des fluides biologiques, notamment des sérums de patients ou d'animaux atteints de Lupus Erythémateux disséminé (LED).

L' invention concerne également les applications de ces peptides et des compositions les contenant pour le diagnostic in vitro chez l'homme du LED, ainsi que leur utilisation à la constitution de trousses ou "kits" de diagnostic.

L'invention concerne en outre les applications de ces peptides à la production de compositions immunogènes et de compositions vaccinantes contre cette maladie.

L'invention concerne enfin les anticorps susceptibles d'être induits in vivo par ces peptides immunogènes ou rendus immunogènes et l'application de ces anticorps et des compositions les contenant pour le diagnostic in vitro chez l'homme atteint de LED, ainsi qu'à la production de médicaments contre cette maladie.

La présence d'autoanticorps dirigés contre les composants cellulaires est la caractéristique générale des maladies autoimmunes telles que le Lupus Erythémateus disséminé (LED), la Sclérodermie (Scl), la Polymyosite et la maladie du tissu connectif (MTCD) (Morrow et Isenberg, 1987; Tan et al., 1988). Parmi les nombreux types d'autoanticorps identifiés dans ces maladies, ceux réagissant avec l'antigène Sm représentent un marqueur très utile car ils sont présents chez 20 à 30 % des sujets atteints de LED et très rarement chez les sujets atteints d'autres maladies autoimmunes systémiques des tissus connectifs.

L'antigène Sm est associé à une classe particulière de ribonucléoprotéines dénommées RNP; ces ribonucléoprotéines contiennent cinq espèces d'ARN riches en uridine dénommés U1, U2, U4, U5 et U6 (Lerner et Steitz, 1979; Brunel et al., 1985), dans chacune desquelles ont été identifiées sept protéines. Ces protéines ont été dénommées, en fonction de leur mobilité électrophorétique sur gels de polyacrylamide, bande B' (29 Kd), bande B (28 Kd), bande D (16 Kd), bande D' (15,5 Kd), bande E (12 Kd), bande F (11 Kd) et bande G (9 Kd). En plus de ces protéines qui constituent le noyau commun, la particule U1-RNA contient trois polypeptides uniques respectivement de 70 Kd, 34 Kd (A) et 22 Kd (C). L'espèce U2-RNP contient deux polypeptides uniques dénommés A' (33 Kd) et B'' (28,5 Kd). Les anticorps anti-RNP ou anti UI-RNP précipitent uniquement les composants de la particule U1, alors que les anticorps anti-Sm réagissent avec les particules U1, U2, U4, U5 et U6.

En outre, des études en immunoblot ont montré que les anticorps anti-U1-RNP réagissent avec les polypeptides A, C et 70 Kd, alors que les anticorps anti-Sm réagissent avec les polypeptides B', B et D; les bandes E, F et G sont également parfois reconnues par les anticorps anti-Sm (Pettersson et al., 1984; Reichlin et Harley; 1987; Hoch, 1989; Combe et al. 1989).

Le séquençage de plusieurs polypeptides des ribonucléoprotéines a été obtenu par des techniques d'ADN recombinant (Theissen et al., 1986; Habets et al., 1987; Stanford et al., 1987; Sillekens et al. 1987, 1989; Yamamoto et al., 1988; Rokeach et al. 1989). La demande de brevet européen N° 295 719 décrit le clonage d'un ADN codant pour l'antigène Sm-D (Rokeach et al., 1988); ce gène code pour un polypeptide de 119 aminoacides contenant plusieurs régions basiques, et les auteurs pensent qu'un motif Glycine-Arginine répété neuf fois et localisé à l'extrémité C-terminale constituerait le déterminant antigénique de l'antigène Sm-D. Cette séquence déduite de l'ADN isolé présente peu de similarité avec les autres polypeptides séquencés précédemment.

La demande de brevet N° 295 719 décrit en outre une méthode de détection du LED en utilisant l'antigène Sm-D cloné.

Les travaux effectués par la demanderesse sur la séquence du polypeptide Sm-D de 119 aminoacides, lui ont permis de constater que certaines séquences peptidiques sélectionnées à partir du dit polypeptide Sm-D présentent un intérêt particulier pour la détection du LED. La demanderesse a remarqué que certains peptides issus du polypeptide Sm-D sont reconnus de manière tout à fait spécifique par des anticorps présents chez les sujets atteint de LED. Lesdits peptides n'étant pas reconnus par les anticorps présents chez les patients atteints de maladies autoimmunes autres que le LED.

Ces observations montrent l'intérêt de ces peptides pour le diagnostic in vitro du LED.

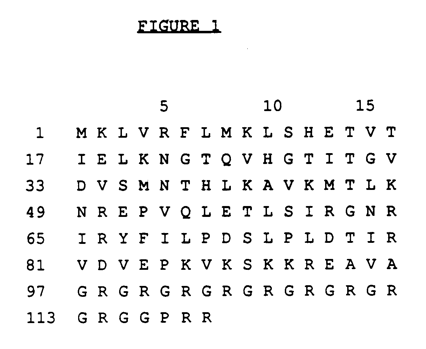

La figure 1 représente la séquence peptidique du polypeptide Sm-D. Les correspondances entre les acides aminés et leur code à une lettre sont les suivantes :
- A: alanine
- C: cystéine
- D: acide aspartique
- E: acide glutamique
- F: phénylalanine
- G: glycine
- H: histidine
- I: isoleucine
- K: lysine
- L: leucine
- M: méthionine
- N: asparagine
- P: proline
- Q: glutamine
- R: arginine
- S: sérine
- T: thréonine
- V: valine
- W: tryptophane
- Y: tyrosine

L'invention concerne des peptides, capables de réagir avec des anticorps contre le polypeptide Sm-D présent dans un échantillon biologique d'un sujet atteint de Lupus Erythemateux Disséminé, ces peptides correspondant à une partie de la séquence du polypeptide Sm-D ou à une variante de cette séquence.

Les peptides préférés selon l'invention sont constitués par l'une des séquences de formules :
XMKLVRFLMKLSHETVTIELKZ (I)
XKMTLKNREPVQLETLSIRGNRIRYZ (IV)
Dans les formules précédentes :
- les groupes X représentent, soit un groupe NH₂ libre, soit un groupe peptidique comprenant de 1 à 5 aminoacides, et
- les groupes Z représentent, soit un groupe OH libre, soit un groupe peptidique de 1 à 5 aminoacides.

Les groupes peptidiques de 1 à 5 aminoacides, éventuellement contenus dans X et/ou Z sont tels que leur présence ne modifie pas essentiellement les propriétés immunologiques, le cas échéant immunogènes, des peptides qui en sont dépourvus, mais peut les accroître.

Des peptides préférés selon l'invention sont les peptides répondant aux séquences de formules (I), et, (IV), dans lesquelles X représente un groupe NH₂ et Z représente un groupe OH ou, dans la mesure où les propriétés immunologiques du peptide ne s'en trouvent pas essentiellement modifiées mais éventuellement augmentées, X et Z représentent chacun un groupe de 1 à 5 aminoacides.

Le peptide I répondant à la formule (I) dans laquelle X est un groupe NH₂ libre et Z un groupe OH libre, correspond aux résidus 1 à 20 du polypeptide Sm-D représenté à la figure 1.

Des peptides préférés répondant à la formule (I) dans laquelle Z représente un groupe de 1 à 5 aminoacides sont, le peptide I prolongé à son extrémité C-terminale par les 1 à 5 aminoacides correspondants dans le polypeptide Sm-D de la figure 1. Ainsi, Z est avantageusement choisi parmi les groupes de 1 à 5 aminoacides suivants : N, NG, NGT, NGTQ, NGTQV.

Le peptide IV répondant à la formule (IV) dans laquelle X est un groupe NH₂ libre et Z un groupe OH libre, correspond aux résidus 44 à 67 du polypeptide Sm-D représenté à la figure 1.

Des peptides préférés répondant à la formule (IV) dans laquelle X et Z représentent chacun un groupe de 1 à 5 aminoacides sont, le peptide IV prolongé de part et d'autre par les 1 à 5 aminoacides correspondants dans le polypeptide Sm-D de la figure 1. Ainsi X est avantageusement choisi parmi les groupes de 1 à 5 aminoacides suivants : V, AV, KAV, LKAV, HLKAV;
et Z est avantageusement choisi parmi les groupes de 1 à 5 aminoacides suivants : F, FI, FIL, FILP, FILPD.

Des peptides de référence II, III et V à VII qui ne font pas partie de l'invention correspondent respectivement aux résidus 17 à 35 (peptide II) ; 33 à 51 (peptide III) ; 64 à 84 (peptide V) ; 77 à 96 (peptide VI) et 97 à 119 (peptide VII) du polypeptide Sm-D représenté à la figure 1. Ainsi X est avantageusement choisi parmi les groupes de 1 à 5 aminoacides suivants : A, VA, AVA, EAVA, REAVA.

On entend par variantes, les séquences des peptides précités modifiées par insertion et/ou délétion et/ou substitution d'un ou plusieurs aminoacides, pour autant que les propriétés antigéniques ou immunogènes desdites séquences ne s'en trouvent pas modifiées; on peut notamment utiliser une partie des peptides précédemment décrit dans la mesure où cette séquence de plus petite taille conserve les propriétés immunologiques, le cas échéant immunogènes, vis à vis des anticorps réagissant avec le polypeptide Sm-D.

On entend aussi par variantes les séquences dans lesquelles la liaison peptidique (-CO-NH-) est remplacée par les structures -CO-N(CH₃)-, -CH₂-CH₂-, - CO-CH₂-, ou encore, les séquences dans lesquelles le squelette peptidique présente un ou plusieurs groupes intercalés tels que les groupes -CH₂-, -NH-, -O-. La présente invention englobe également les peptides dans lesquels les aminoacides présentant un carbone asymétrique sont sous forme D ou L.

L'invention concerne également les conjugués obtenus par couplage des peptides de l'invention avec des molécules porteuses éventuellement physiologiquement acceptables et non toxiques; à titre de molécules porteuses on peut citer des protéines naturelles comme l'anatoxine tétanique, l'albumine ou des sérums albumines.

Les peptides de l'invention possèdent des propriétés antigèniques et peuvent donc être utilisés dans des procédés de diagnostic pour la détermination ou le suivi de patients atteints de LED. L'invention concerne donc également les compositions contenant les peptides précédents ou un mélange de ces peptides ou encore ces peptides conjugués à une molécule porteuse, et susceptibles d'être reconnus par les autoanticorps présents dans le sérum ou tout autre fluide biologique de patients atteints de LED. La détection du complexe peptide-anticorps in vitro est effectuée par des tests immunoenzymatiques du type ELISA, d'immunofluorescence, radioimmunologiques ou de radioimmunoprécipitation, ou d'immunotransfert (Immunoblot ou de dot-blot).

Pour la mise en oeuvre de ces tests, l'invention concerne les peptides froids non marqués ou marqués à l'aide d'un marqueur adéquat qui peut être de la biotine ou ses dérivés, une enzyme comme la peroxydase, un marqueur fluorescent comme la fluorescéine, un marqueur radiactif, etc...

De tels tests comprennent par exemple les étapes suivantes :
- dépôt d'une quantité déterminée d'une composition contenant un peptide ou un conjugué d'un peptide selon l'invention, dans les puits d'une plaque de microtitration ou sur un autre support tel que des billes ou des membranes de nitrocellulose, par exemple,
- dépôt dans les puits du liquide biologique à tester ou incubation de celui-ci avec les billes ou la membrane, en présence d'agents saturant ou après saturation préalable des supports activés,
- après incubation et rinçage des microplaques ou des billes, dépôt dans les puits ou incubation avec les billes d'un système de révélation du complexe peptide-anticorps éventuellement formé.

Dans un autre mode de réalisation de ce type de test, on met en oeuvre un mélange de peptides reconnaissant d'une part des anticorps présents dans le sérum de patients atteints de LED et d'autre part des anticorps présents dans des sérums de patients atteints d'une autre maladie autoimmune.

L'invention concerne encore les anticorps formés contre les peptides de l'invention. Anticorps qui peuvent être polyclonaux, ou monoclonaux et produits alors par tout hybridome préparé selon les méthodes classiques de fusion cellulaire entre des cellules spléniques activées in vitro par l'antigène ou provenant d'un animal immunisé contre l'un des peptides de l'invention et des cellules d'une lignée de cellule myélomateuse.

En raison de la spécificité des peptides de l'invention vis à vis des anticorps des patients atteints de LED, les anticorps préparés à partir de ces peptides constituent des sondes également très spécifiques de l'antigène Sm-D du LED.

Par ailleurs, les anticorps formés contre les peptides de l'invention et les autoanticorps des patients réagissant avec lesdits peptides et obtenus après chromatographie d'affinité peuvent être utilisés pour préparer des anticorps anti-idiotypes constituant en partie une copie exacte du peptide antigènique initial et donc capable de se lier aux autoanticorps observés chez les patients atteints de LED.

La présente invention concerne donc ces anticorps anti-idiotypes et les compositions les contenant ainsi que leur application pour le diagnostic in vitro chez l'homme de la présence d'autoanticorps observés dans le cas de LED.

L'invention concerne enfin des compositions immunogènes pour la production de vaccins dont le principe actif est constitué par au moins un peptide ou un anticorps anti-idiotype selon l'invention, éventuellement conjugué à une molécule porteuse, induisant la production d'anticorps contre lesdits peptides et qui sont capables d'interférer avec la pathologie et/ou les manifestations cliniques du LED. Les compositions pharmaceutiques selon l'invention, utilisables comme vaccin sont constituées par des solutions ou suspensions injectables ou administrables par d'autres voies pouvant être administrées à des doses situées entre 10 µg/Kg et 100 mg/Kg de peptide selon l'invention.

D'autres caractéristiques et avantages de l'invention apparaîtront à la lecture des exemples qui suivent et qui sont illustrés par les figures en annexe, étant entendu que ces exemples ne sauraient être interprétés comme tendant à réduire la portée des revendications.

### EXEMPLE 1

### SYNTHESE, PURIFICATION ET CARACTERISATION DES PEPTIDES

Les peptides selon l'invention peuvent être préparés par les techniques classiques de synthèse peptidique en phase solide, soit par condensation successive des résidus d'acides aminés dans l'ordre requis, soit par condensation des résidus d'acides aminés sur un fragment préalablement formé et contenant déjà plusieurs aminoacides dans l'ordre approprié ou encore par condensation de plusieurs fragments préalablement préparés, en prenant soin de protéger au préalable toutes les fonctions réactives portées par les résidus d'acides aminés ou les fragments, exceptées les fonctions amine et carboxyle engagées dans la liaison peptidique formée lors de la condensation.

Selon un mode de préparation préféré des peptides de l'invention, on utilise une résine PAM; la fonction amine de l'acide aminé ajouté est protégée par un groupe terbutyloxycarbonyle (Boc), les chaînes latérales des acides aminés trifonctionnels sont protégées par exemple par les groupes suivants : le cyclohexyle pour l'acide glutamique et l'acide aspartique, le benzyle pour la thréonine et la sérine, le 2-chlorobenzyloxycarbonyle pour la lysine, le 2,6-dichlorobenzyle pour la tyrosine, le p-toluènesulfonyle pour l'arginine et l'histidine. La methionine est introduite sous forme de terbutyloxycarbonyle (O) sulfoxyde methionine qui est réduite en méthionine lors du clivage final du peptide de la résine par action de l'acide fluorhydrique anhydre. Les bocs acides aminés sont couplés sous forme d'ester de benzotriazole sauf Boc histidine qui est introduit sous forme d'anhydre symetrique.

Le temps de couplage total est d'environ 45 minutes; un test à la ninhydrine est utilisé pour vérifier la bonne réalisation du couplage lequel, si nécessaire, peut être doublé.

A la fin de la synthèse et après la dernière étape de déprotection, la résine est lavée et sèchée. Selon la présence ou l'absence de (O)méthionine dans la séquence un traitement fort ou faible à l'acide fluorydrique est utilisé pour la déprotection et le clivage du peptide de la résine.

Après lyophilisation le produit brut est dissout dans l'acide acétique 10 % et purifié par chromatographie moyenne pression. Les peptides sont alors caractérisés par chromatographie en phase liquide à haute pression ainsi que par l'analyse de leur composition en aminoacides par spectrométrie de masse.

### EXEMPLE 2

### ETUDES SEROLOGIQUES

### 1 - Matériels

a - 165 patients atteints d'un lupus érythémateux disséminé (LED) actif ont été testés et comparés à ceux de 32 patients atteints de Sclerodermi (Scl), de 14 patients atteints de maladie mixte du tissu connectif (MTCD), de 2 atteints de Polymyosite, de 13 patients atteints de Sarcoidose, de 5 patients atteints du syndrome de Sjogren, de 35 patients atteints d'arthrite rhumatoïde (RA) et de 86 patients atteints d'arthrite chronique juvénile (JCA).
b - 53 sérums de volontaires sains ont été utilisés comme témoins.
c - 18 sérums humains et 5 anticorps monoclonaux de souris reconnaissant en immunoblot les bandes de l'antigène Sm ont également été utilisés. Ces 5 anticorps proviennent respectivement du clone H126 (décrit par Reuter et Lührmann en 1986) réagissant avec les bandes B', B et D, du clone Y12 (décrit par Pettersson et al. en 1984) réagissant avec les bandes B', B, D et E, du clone H57 (décrit par R. Lührmann) réagissant avec les bandes B1 et B, et des clones 2-73 (anti-U1 RNP) et 7-13 (anti-Sm) (décrits par Billings et al. en 1982 et 1985) qui lient le polypeptide D.
d - les peptides I, II, III, IV, V, VI et VII ont été synthétisés selon la technique décrite à l'exemple I, leur degré de pureté est d'au moins 85 %.

### 2) Méthode

La réaction immunologique entre les peptides I, II, III, IV, V, VI et VII et les anticorps des différents sérums a été mesurée par ELISA selon le protocole suivant :
- 1 à 2 µM de chaque peptide dilué dans une solution 0,05 M de tampon carbonate à pH 9,6 sont déposés dans les puits d'une plaque de microtitration, à une température de 37° Celsius; les plaques sont alors misent à incuber 1 heure à 37° Celsius avec une solution à 10mg/ml de sérum albumine bovine (BSA) en tampon PBS pH 7,4 contenant 0,05 % de Tween 20 (PBS-T-BSA);
- après trois lavages avec le tampon PBS-T, le sérum du patient dilué au 1/1000 dans le tampon PBS-T-BSA, est ajouté dans les puits et laissé incuber 1 heure à 37° Celsius;
- après plusieurs lavages, la réaction est détectée par incubations successives d'une heure à 37° Celsius avec un conjugué de biotine spécifique de l'IgG humaine, puis avec un conjugué streptavidine marqué à la peroxydase également pendant 1 heure à 37° Celsius.

La révélation de la réaction enzymatique est effectuée par addition d'un substrat de la peroxydase tel que le 2,2'-azinobis(3-ethylbenzthiazoline sulfonate) (ABTS) pendant une heure à 37° Celsius. La valeur de la densité optique (DO) est mesurée au spectrophotomètre à 405 nm. La révélation peut également se faire en une seule étape après incubation du sérum en ajoutant un conjugué anti IGg humaine marqué à la peroxydase (30 mn à 37° Celsius) puis le substrat (TMB 3, 3', 5' tétra méthyl benzidine) pour 15 mn à 37° Celsius). La réaction est bloquée par addition d'HCl 2M et la DO est lue à 450 nm.

Afin de déterminer le seuil de positivité du test, une série de 53 sérums d'individus en bonne santé, dilués au 1/1000 ont été testés avec 1 uM et 2 uM des peptides I et IV disposés dans les puits de microplaques; l'absorption moyenne à 405 nm est de 0,10 avec une déviation standard de 0,084. Un sérum est donc considéré comme positif lorsque la valeur de sa DO est supérieure à la moyenne additionnée de deux fois la déviation standard, c'est-à-dire 0,3 unité de DO.

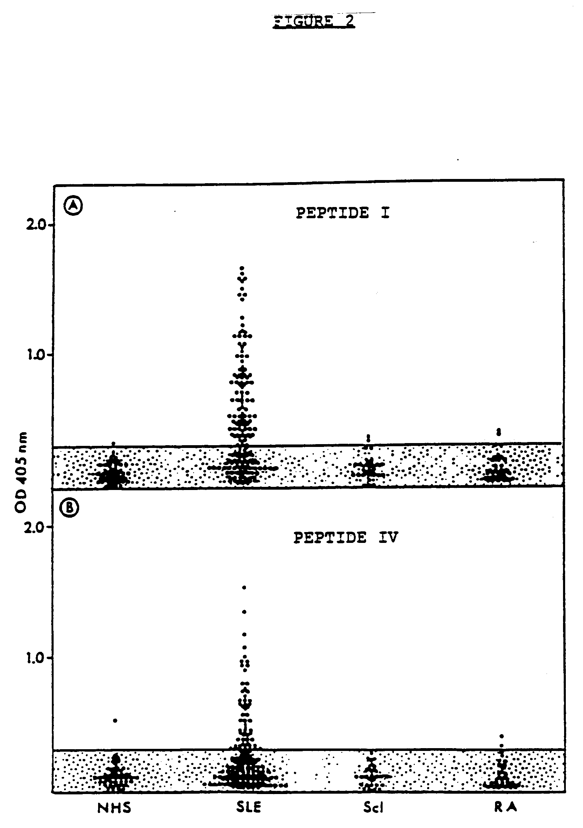

En utilisant ce seuil, 1,8 % des sérums d'individus normaux ont été trouvés positifs avec les deux peptides (Figure 2). En mettant le seuil à DO moyenne plus 5 déviations standards le test devient totalement spécifique pour le LED.

### 3) Etudes des 18 sérums de patients reconnaissant la bande D en Immunoblot

Parmi ces sérums, 12 soit 67 %, ont réagi avec le peptide I (DO comprise entre 0,30 et 0,81; valeur moyenne de la DO 0,47 et valeur de la déviation standard 0,15), 16 soit 89 %, ont réagi avec le peptide IV (DO comprise entre 0,30 et 1,20; valeur moyenne de la DO 0,57 et valeur de la déviation standard 0,25) et 6 soit 33 %, ont réagi avec le peptide VII (DO comprise entre 0,30 et 0,73; valeur moyenne de la DO 0,46 et valeur de la déviation standard 0,16).

Tous les sérums ayant réagi avec le peptide I et/ou le peptide VII ont également réagi avec le peptide IV. Aucun des sérums n'a réagi avec les peptides II, III, V et VI.

Il apparaît donc que la majeure partie des sérums réagissant en immunoblot avec la bande D peuvent être identifiés par leur réaction en ELISA avec le seul peptide IV.

En outre, les cinq anticorps monoclonaux de souris réagissant en immunoblot avec certaines des bandes de l'antigène Sm ont été testés avec les peptides I, II, III, IV, V, VI et VII.

Les anticorps des clones 2-73 et 7-13 ont réagi avec avec le peptide VII en ELISA. Les trois autres anticorps n'ont réagi avec aucun des sept peptides.

### 4) Etude des sérums des patients atteints de LED et d'autres maladies rhumatiques

Parmi les 165 patients atteints de LED, 59 % possèdent des anticorps pouvant réagir avec le peptide I (valeur de la DO comprise entre 0,30 et 1,67; valeur moyenne de la DO 0,77), 37 % d'entre eux possèdent des anticorps réagissant avec le peptide IV (valeur de la DO comprise entre 0,30 et 0,58; valeur moyenne de la DO 0,58), deux sérums hors des 165 testés ont réagi avec le peptide VII, et aucun sérum n'a réagi avec les peptides II, III, V et VI (Figure 2, tableau I).

Les peptides I et IV sont rarement reconnus par les anticorps des sérums de patients atteints d'une autre maladie rhumatismale que le LED. 6 % des sérums de patients atteints de RA ont réagi avec l'un des peptides I (valeur de la DO comprise entre 0,30 et 0,39) et IV (valeur de la DO comprise entre 0,30 et 0,41). Seulement 6 % des sérums de patients atteints de Scl ont réagi avec le peptide I (valeur de la DO comprise entre 0,30 et 0,36) et aucun avec le peptide IV.

Les résultats concernant les peptides I et IV sont rapportés dans le tableau I ci-dessous. Les sérums des patients ont été dilués au 1/1000 et mis en contact avec 1 uM du peptide I et 2 uM du peptide IV adsorbés sur une plaque de microtitration. Les valeurs sont exprimées en pourcentage de sérums positifs par rapport au nombre total de sérums testés. Un sérum est considéré positif lorsque la valeur de la DO est supérieure ou égale à 0,3 (ce qui correspond à la valeur moyenne de la DO pour les 53 sérums d'individus en bonne santé ajouté de de deux fois la déviation standard) après une heure d'incubation de l'anti-IgG humaine conjugué à l'enzyme avec le substrat de ladite enzyme.

**TABLEAU I**

| type de sérum | nombre de sérums | peptide I | peptide IV |
|---|---|---|---|
| LED | 165 | 58,8 % | 37,0 % |
| Scl | 32 | 6,3 | 0 |
| MCTD | 14 | 0 | 0 |
| Polmyosite | 2 | 0 | 0 |
| Sarcoidose | 13 | 0 | 0 |
| Syndrome de Sjögren | 5 | 0 | 0 |
| RA | 35 | 5,7 | 5,7 |
| JCA | 86 | 1,2 | 1,2 |
| Normal | 53 | 1,8 | 1,8 |

Ces résultats sont également rapportés à la figure 2 en annexe selon une autre présentation.

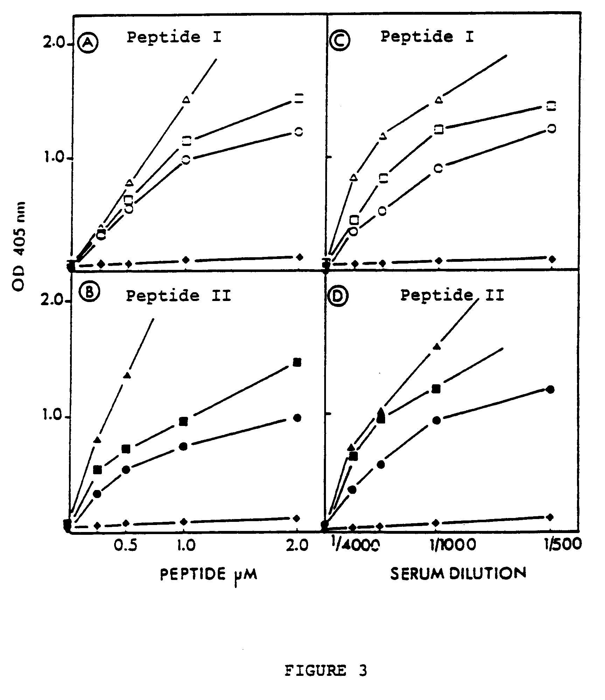

Dans la mesure où tous les sérums positifs avec le peptide IV sont également positifs avec le peptide I en ELISA, l'utilisation du peptide I en ELISA permet d'identifier environ 60 % des sérums de patients atteints de Lupus Erythemateux Disséminé. La figure 3 en annexe montre la liaison des autoanticorps présents dans le sérum de patients atteints de LED avec les peptides I et IV.

### EXEMPLE 3

### PREPARATION ET ETUDE D'ANTISERUMS OBTENUS CHEZ LE LAPIN

### 1) Préparation des antisérums

Des antisérums contre les peptides I, II, III, IV, V, VI et VII sont obtenus chez le lapin. A chaque injection le lapin recoit 100 ug de peptide dans une solution saline en présence de l'adjuvant de Freund (V/V). Des séries d'injections sous cutanés sont administrées deux fois par mois pendant quatre mois en utilisant deux lapins par peptide.

Après trois injections, le sang des lapins est régulièrement prélevé une semaine après chaque injection et le taux d'anticorps sériques est mesuré en ELISA.

### 2) Méthode

La liaison des anticorps de lapin avec les 7 peptides est mesurée en ELISA selon une méthode identique à celle décrite précédemment (voir EXEMPLE II point 2), excepté en ce qui concerne la réaction immunologique qui est révélée par addition d'anti-immunoglobulines de lapin préparées chez la chèvre et conjuguées à de la peroxydase.

En outre, pour tester les anticorps monoclonaux, les surnageants des cultures d'hybridomes sont dilués au 1/50 dans un tampon PBS-T-BSA et mis en contact avec les peptides. La réaction est révélée par addition successive d'anti-immunoglobulines de souris préparées chez le lapin et d'anti-immunoglobulines de lapin préparées chez la chèvre et conjuguées à la peroxydase comme précédemment.

### 3) Résultats

Une forte réponse est obtenue pour les peptides I, II, III, IV, V et VI après trois injections aux lapins (la valeur de la DO à 405 nm est supérieure à 2,0 après 60 minutes d'incubation du substrat avec les antisérums dilués au 1/5000 et 1/10000.

Ces résultats indiquent que l'incapacité des peptides II, III, V et VI à réagir avec le sérum des patients, voir tableau I ci-dessus, n'est pas due à une mauvaise adsorption de ces peptides sur la plaque ELISA. Seul le peptide VII donne une réponse plus modérée chez les lapins (la valeur de la DO est de 2,0 après 60 minutes d'incubation avec les antisérums dilués au 1/250 et 1/500). Cette réactivité relativement faible de l'antisérum avec le peptide VII n'est pas due à une mauvaise adsorption du peptide sur la plaque ELISA puisque les anticorps monoclonaux 2-73 et 7-13 ont réagi fortement avec ce peptide, (voir EXEMPLE II point 3).

### EXEMPLE IV

### ETUDE DE LA CORRELATION ENTRE LED ANTICORPS ANTI-ADN ET LES ANTICORPS CONTRE LES PEPTIDES DU POLYPEPTIDE D

Les sérums des patients atteints de LED ont également été testés pour déterminer la présence d'anticorps réagissant en ELISA avec de l'ADN natifs double brin. Aucune corrélation n'a été mise en évidence entre la présence d'anticorps anti-ADN et la présence d'anticorps réagissant avec les peptides I ou IV.

### EXEMPLE V

### PREDICTION DES SITES ANTIGENIQUES DE L'ANTIGENE Sm-D

La méthode mise en oeuvre utilise des algorithmes basés sur certains paramètres comme l'hydrophilie et la mobilité de courts segments de la structure primaire des protéines (Van Regenmortel et Daney de Marcillac, 1988).

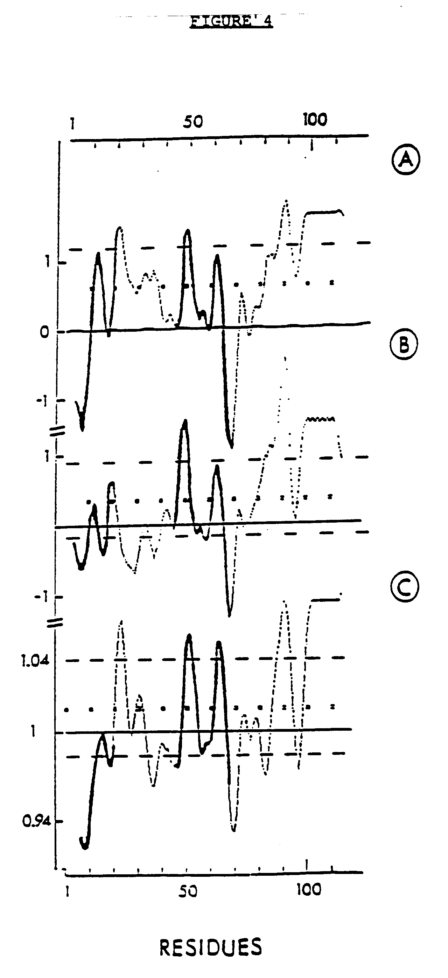

De manière surprenante les résultats obtenus n'indiquent pas que le peptide I, s'étendant des résidus 1 à 20 du polypeptide Sm-D, comprend un épitope déterminant du polypeptide. Ces résultat sont rapportés à la figure 4.

Les légendes des figures illustrant la description qui précèdent sont les suivantes :
- La figure 1 représente la séquence déduite du polypeptide Sm-D selon le c-DNA.
- La figure 2 représente la liaison en ELISA des peptides I et IV. L'activité des IgG anti-peptide est mesurée sur 53 sérums de sujets en bonne santé (NHS), 165 sérums de patients atteints de Lupus Erythémateux disséminé (LED), 32 sérums de patients atteints de Sclerodermie (Scl) et 35 sérums de patients atteints d'Arthrite Rhumatoïde (RA). Tous les sérums ont été dilués au 1/1000 et le niveau d'anticorps est exprimé en unité de densité optique (OD) à 405 nm après hydrolyse du substrat pendant 60 minutes.
- La figure 3 représente la liaison en ELISA de 6 sérums de patients atteints de Lupus Erythémateux disséminé (**l**,**n**,**s**,**m**,**r**,**s**), et de 1 sérum de sujet en bonne santé (**u**), avec le peptide I (cadres A et C) et le peptide IV (cadres B et D). En A et B, les sérums sont dilués au 1/1000 et mis en contact avec diverses concentrations du peptide I; en B et D, diverses concentrations des sérums sont mises en contact respectivement avec 1 et 2 µM des peptides I et IV.
- La figure 4 représente les profiles de prédiction antigènique du polypeptide Sm-D construits selon les échelles suivantes :
   . en A : selon Parker et al. (1986),
   . en B : selon Hopp et Woods (1981),
   . en C : selon Karplus et Schultz (1985),
ces échelles ont été normalisées selon Van Regenmortel et Daney de Marcillac (1988). Les graphiques sont tracés entre le quatrième résidu et le résidu n-3. Les épitopes reconnus par les anticorps des patients atteints de LED sont représentés en trait épais.

L'ensemble des résultats énoncés dans les exemples précédents indique clairement que les peptides issus de l'antigène Sm-D sont particulièrement utiles pour la mise en oeuvre d'un test de diagnostic quantitatif d'une grande sensibilité du LED.

Les observations recueillies sur le peptide I montrent que 59% des patients atteints de LED possèdent des anticorps du type IgG reconnaissant le peptide, alors que 6% des sérums de patients atteints d'une autre maladie autoimmune et moins de 4 % des sérums de sujets normaux réagissent avec le peptide I en ELISA. Le peptide I, éventuellement utilisé avec le peptide IV, constitue donc une sonde particulièrement efficace pour le diagnostic du Lupus Erythémateux Disséminé.

Ce pourcentage de près de 60% est supérieur à ce qui a été décrit antérieurement avec des protéines purifiées.

### REFERENCES

Billings, P.B., Allen, R.W., Jensen, F.C. and Hoch, S.O. 1982. Anti-RNP monoclonal antibodies derived from a mouse strain with lupus-like autoimmunity. *J. Immunol.* 128: 1176.

Billings, P.B., Barton, J.R. and Hoch, S.O. 1985. A murine monoclonal antibody recognizes the 13,000 molecular weight polypeptide of the Sm small nuclear ribonucleoprotein complex. *J. Immunol.* 135: 428.

Bringmann, P. and Lührmann, R. 1986. Purification of the individual snRNPs U1, U2, U5 and U4/U6 from HeLa cells and characterization of their protein constituents. *The Embo J.* 5: 3509.

Brunel, C., Sri-Widada, J. and Jeanteur, P. 1985. snRNP's and scRNP's in eukaryotic cells. In *Progr. Mol. Subcell. Biol. Vol.9.* F.E. Hahn, D.J. Kopecko, W.E.G. Müller, eds. Springer Verlag (Berlin), p. 1-52.

Combe, B., Rucheton, M., Graafland, H., Lussiez, V., Brunel, C. and Sany, J. 1989. Clinical significance of anti-RNP and anti-Sm autoantibodies as determined by immunoblotting and immunoprecipitation in sera from patients with connective tissue diseases. *Clin. Exp. Immunol.* 75: 18.

Eisenberg, R.A., Dyer, K., Craven, S.Y., Fuller. C.R. and Yount, W.J. 1985. Subclass restriction and polyclonality of the systemic lupus erythematosus marker antibody anti-Sm. *J. Clin. Invest.* 75: 1270.

Habets, W.J., Sillekens, P.T.G., Hoet, M.H., Schalken, J.A., Roebroek, A.J.M., Leunissen, J.A.M., Van de Ven, W.J.M. and Van Venrooij, W.J. 1987. Analysis of a cDNA clone expressing a human autoimmune antigen : Full-length sequence of the U2 small nuclear RNA-associated B'' antigen. *Proc. Natl. Acad. Sci. USA* 84: 2421.

Hardin, J.A. 1986. The lupus autoantigens and the pathogenesis of systemic lupus erythematosus. *Arthritis Rheum.* 29: 457.

Hoch, S.O. 1989. Application of protein blotting to the study of autoimmune disease. In*Protein Blotting. Methodology, research and diagnostic applications.* B.A. Baldo, E.R. Tovey, S. Karger AG, Basel, p. 140-164.

Hopp, T.P. and Woods, K.R. 1981. Prediction of protein antigenic determinants from amino acid sequences. *Proc. Natl. Acad. Sci. USA* 78: 3824.

Kaiser, E., Colescott, R.L., Bossinger, C.D. and Cook, P.I. 1970. Color test for detection of free terminal amino groups in the solid phase synthesis of peptides. *Anal. Biochem.* 34: 595.

Karplus, P.A. and Schulz, G.E. 1985. Prediction of chain flexibility in proteins. *Naturwissenschaften* 72:212.

Lerner, M.R. and Steitz, J.A., 1979. Antibodies to small nuclear RNAs complexed with proteins are produced by patients with systemic lupus erythematosus. *Proc. Natl. Acad. Sci. USA* 76: 5495.

McCarty, G.A., Rice, J.R., Bembe, M.L. and Pisetsky. D.S. 1982. Independent expression of autoantibodies in systemic lupus erythematosus. *J. Rheum* 9: 691.

Merrifield, R.B. 1963. Solid phase peptide synthesis. I. The synthesis of a tetrapeptide. *J. Amer. Chem. Soc.* 85: 2149.

Morrow, J. and Isenberg, D.A., 1987. Systemic Lupus Erythematosus. In *Autoimmune Rheumatic Diseases,* chap. 3. Blackwell Scientific Publ., pp. 48.

Muller, S., Plaué, S., Couppez, M. and Van Regenmortel, M.H.V. 1986. Comparison of different methods for localizing antigenic regions in histone H2A. *Molec. Immunol.* 23: 593.

Muller, S., Bonnier. D., Thiry, M. and Van Regenmortel, M.H.V. 1989. Reactivity in systemic lupus erythematosus with synthetic core histone peptides. *Int. Arch. Allergy Appl. Immunol.* 89: 288.

Muller, S., Briand, J.P. and Van Regenmortel, M.H.V., 1988. presence of antibodies to ubiquitin during the autoimmune response associated with systemic lupus erythematosus. *Proc. Natl. Acad. Sci. USA* 85: 8176.

Parker, J.M.R., Guo, D. and Hodges, R.S., 1986. New hydrophilicity scale derived from high-performance liquid chromatography peptide retention data : Correlation of predicted surface residues witn antigenicity and X-ray-derived accessible sites. *Biochemistry* 25: 5425.

Pettersson, I., Hinterberger, M., Mimori, T., Gottlieb, E. and Steitz, J.A., 1984. The structure of mammalian small nuclear ribonucleoproteins*. J. Biol. Chem.* 259: 5907.

Plaué, S. and Briand, J.P., 1988. Solid-phase peptide synthesis. In *Synthetic Polypeptides as Antigens* In the series *Laboratory Techniques in Biochemisty and Molecular Biology, vol. 19.* R.H. Burdon and P.H. Van Knippenberg, eds.Elsevier, Amsterdam, p. 41-94.

Plaué, S., Muller, S. and Van Regenmortel, M.H.V. 1989. A branched, synthetic octapeptide of ubiquinated histone H2A as target of autoantibodies. *J. Exp. Med.* 169: 1607.

Reichlin, M. and Harley, J.B. 1987. ANA subsets in systemic lupus erythematosus. In *Systemic Lupus Erythematosus.* J.S. Smolen, C.C. Zielinski, eds. Springer Verlag (Berlin), p. 105-123.

Reuter. R. and Lührmann. R. 1986. Immunization of mice with purified U1 small nuclear ribonucleoprotein (RNP) induces a pattern of antibody specificities characteristic of the anti-Sm and anti-RNP autoimmune response of patients with lupus erythematosus, as measured by monoclonal antibodies*. Proc. Natl. Acad. Sci. USA* 83: 8689.

Rokeach, L.A., Haselby, J.A. and Hoch, S.O. 1988. Molecular cloning of a cDNA encoding the human Sm-D autoantigen. *Proc. Natl. Acad. Sci. USA* 85: 4832.

Rokeach, L.A., Jannatipour, M., Haselby, J.A. and Hoch, S.O. 1989. Primary structure of a human small nuclear ribonucleoprotein polypeptide as deduced by cDNA analysis. *J. Biol. Chem.* 264: 5024.

Sillekens, P.T.G., Habets W.J., Beijer, R.P. and Van Venrooij, W.J. 1987. cDNA cloning of the human U1 snRNA-associated A protein : extensive homology between U1 and U2 snRNP-specific proteins. *The EMBO J.* 6: 3841.

Sillekens, P.T.G., Beijer, R.P., Habets, W.J. and Van Venrooij, W.J. 1989. Molecular cloning of the cDNA for the human U2 snRNA-specific A' protein. *Nucl. Acids Res.* 17: 1893.

Stanford, D.R., Rohleder, A., Neiswanger, K. and Wieben, E.D. 1987. DNA sequence of a human Sm autoimmune antigen. *J. Biol. Chem.* 262: 9931.

Tam, J.P., Heath, W.F. and Merrifield, R.B. 1983. S_{N}2 deprotection of synthetic peptides with a low concentration of HF in dimethyl sulfide : evidence and application in peptide synthesis. *J. Amer. Chem. Soc.* 105: 6442.

Tan, E.M., Chan, E.K.L., Sullivan, K.F. and Rubin, R.L. 1988. Antinuclear antibodies (ANAs) : Diagnostically specific immune markers and clues toward the understanding of systemic autoimmunity. *Clin. Immunol. Immunopath.* 47: 121.

Tan, E.M. and Kunkel, H.G. 1966. Characteristics of a soluble nuclear antigen precipitating with sera of patients with systemic lupus erythematosus. *J. Immunol.* 96: 464.

Theissen. H., Etzerodt, M., Reuter, R., Schneider, C.. Lottspeich, F., Argos, P., Lührmann, R. and Philipson, L. 1986. Cloning of the human cDNA for the U1 RNA-assosiated 70K protein. *The EMBO J.* 5: 3209.

Tuaillon, N., Muller, S., Pasquali, J.L., Bordigoni, P., Youinou, P., and Van Regenmortel, M.H.V. 1990. Antibodies from patients with rheumatoid arthritis and juvenile chronic arthritis analyzed with core histone synthetic peptides. Int. Arch. All. Appl. Immunol. (in press).

Van Regenmortel, M.H.V. and Daney de Marcillac,. G. 1988. An assesment of prediction methods for locating continuous epitopes in proteins. *Immunol. Lett.* 17: 95.

Williams D.G., Charles, P.J. and Maini, R.N., 1988. Preparative isolation of p67, A, B, B' and D from nRNP/Sm and Sm antigens by reverse-phase chromatography. *J. Immunol. Meth.* 113: 25.

Yamamoto, K., Miura, H., Moroi, Y., Yoshinoya, S., Goto, M., Nishioka, K. and Miyamoto, T. 1988. Isolation and characterization of a complementary DNA expressing human U1 small nuclear ribonucleoprotein C Polypeptide. *J. Immunol.* 140: 311.

## Revendications (Revendications pour l'(les) Etat(s) contractant(s) suivant(s): AT, BE, CH, DE, DK, FR, GB, GR, IT, LI, LU, NL, SE)

1. Peptide capable de réagir avec des anticorps contre le polypeptide Sm-D présents dans un échantillon biologique d'un sujet atteint de Lupus Erythémateux Disséminé, ce peptide étant choisi parmi
(1) un peptide de la séquence :
XMKLVRFLMKLSHETVTIELKZ (I)
dans laquelle,X représente un groupe NH₂ libre, et Z représente soit un groupe OH libre soit un groupe peptidique choisi parmi les groupes de 1 à 5 aminoacides suivants : N, NG, NGT, NGTQ, NGTQV;
et (2) un peptide de la séquence :
XKMTLKNREPVQLETLSIRGNRIRYZ (IV)
dans laquelle X représente, soit un groupe NH₂ libre soit un groupe peptidique choisi parmi les groupes de 1 à 5 aminoacides suivants : V, AV, KAV, LKAV, KLKAV;
et Z représente, soit un groupe OH libre soit un groupe peptidique choisi parmi les groupes de 1 à 5 aminoacides suivants : F, FI, FIL, FILP, FILPD.

2. Anticorps dirigés contre l'antigène Sm-D, caractérisés en ce qu'ils sont reconnus par les peptides selon la revendication 1.

3. Anticorps anti-idiotypes formés contre un anticorps selon la revendication 2, caractérisés en ce qu'ils reconnaissent, de manière spécifique, les autoanticorps présents dans des fluides biologiques, notamment des sérums, de patients atteints de Lupus Erythémateux Disséminé.

4. Composition antigénique contenant au moins un peptide selon la revendication 1, caractérisée en ce qu'elle réagit immunologiquement avec les autoanticorps présents dans des fluides biologiques, notamment des sérums de patients atteints de Lupus Erythémateux Disséminé.

5. Composition immunogène contenant au moins un peptide selon la revendication 1, ou un conjugué de ce peptide avec une molécule porteuse, caractérisée en ce qu'elle induit la production d'anticorps capables de reconnaître l'antigène Sm-D.

6. Procédé de diagnostic in vivo du Lupus Erythémateux Disséminé dans un fluide biologique, caractérisé en ce qu'il comprend au moins les étapes suivantes :
- la mise en contact de ce fluide biologique avec au moins un peptide selon la revendication 1, ou un conjugué de ce peptide avec une molécule porteuse, ou encore un anticorps antiidiotype selon la revendication 3, dans des conditions permettant la formation d'un complexe immunologique;
- la détection de la présente d'un complexe immunologique antigène-anticorps ou d'un complexe anticorps-anticorps anti-idiotype, par des méthodes physiques ou chimiques, dans ledit fluide biologique.

7. Kit pour le diagnostic in vitro du Lupus Erythémateux Disséminé, caractérisé en ce qu'il comprend:
- au moins un peptide selon la revendication 1, ou un conjugué de ce peptide avec une molécule porteuse, ou encore un anticorps anti-idiotype selon la revendication 3;
- des réactifs pour la constitution d'un milieu propice à la réaction immunologique entre les peptides ou les anticorps anti-idiotypes et les autoanticorps éventuellement présents dans un échantillon biologique;
- un ou plusieurs réactifs éventuellement marqués aptes à réagir avec le ou les peptides ou les anticorps anti-idiotype pour la détection du complexe immunologique formé;
- le cas échéant, un milieu biologique de référence, tel qu'un sérum d'un patient d'un sujet en bonne santé.

## Revendications (Revendications pour l'(les) Etat(s) contractant(s) suivant(s): ES)

1. Procédé de préparation d'un peptide capable de réagir avec des anticorps contre le polypeptide Sm-D présents dans un échantillon biologique d'un sujet atteint de Lupus Erythémateux Disséminé, ce peptide étant choisi parmi
(1) un peptide de la séquence :
XMKLVRFLMKLSHETVTIELKZ (I)
dans laquelle X représente un groupe NH₂ libre, et Z représente soit un groupe OH libre soit un groupe peptidique choisi parmi les groupes de 1 à 5 aminoacides suivants : N, NG, NGT, NGTQ, NGTQV;
et (2) un peptide de la séquence :
XKMTLKNREPVQLETLSIRGNRIRYZ (IV)
dans laquelle X représente, soit un groupe NH₂ libre, soit un groupe peptidique choisi parmi les groupes de 1 à 5 aminoacides suivants : V, AV, KAV, LKAV, KLKAV;
et Z représente, soit un groupe OH libre soit un groupe peptidique choisi parmi les groupes de 1 à 5 aminoacides suivants : F, FI, FIL, FILP, FILPD :
- soit par synthèse peptidique en phase solide,
- soit par condensation des résidus d'amino acides dans l'ordre approprié,
- soit par condensation de plusieurs fragments préalablement préparés, les fonctions réactives portées par les résidus d'aminoacides ou les fragments à l'exception des fonctions amino et carboxyle engagées dans la liaison peptidique étant protégées au préalable.

2. Procédé de préparation d'anticorps dirigés contre l'antigène Sm-D, reconnus par les peptides selon la revendication 1, caractérisés en ce qu'ils sont obtenus à l'aide d'un hybridome ou par immunisation d'un animal contre l'un desdits peptides définis à la revenidication 1.

3. Procédé de préparation d'anticorps anti-idiotypes formés contre un anticorps selon la revendication 2, caractérisés en ce qu'ils reconnaissent, de manière spécifique, les autoanticorps présents dans des fluides biologiques, notamment des sérums, de patients atteints de Lupus Erythémateux Disséminé.

4. Procédé de préparation d'une composition antigénique contenant au moins un peptide selon la revendication 1, caractérisée en ce qu'elle réagit immunologiquement avec les autoanticorps présents dans des fluides biologiques, notamment des sérums de patients atteints de Lupus Erythémateux Disséminé.

5. Procédé de préparation d'une composition immunogène contenant au moins un peptide selon la revendication 1, ou un conjugué de ce peptide avec une molécule porteuse, caractérisée en ce qu'elle induit la production d'anticorps capables de reconnaître l'antigène Sm-D.

6. Procédé de diagnostic in vivo du Lupus Erythémateux Disséminé dans un fluide biologique, caractérisé en ce qu'il comprend au moins les étapes suivantes :
- la mise en contact de ce fluide biologique avec au moins un peptide selon la revendication 1, ou un conjugué de ce peptide avec une molécule porteuse, ou encore un anticorps antiidiotype selon la revendication 3, dans des conditions permettant la formation d'un complexe immunologique;
- la détection de la présente d'un complexe immunologique antigène-anticorps ou d'un complexe anticorps-anticorps anti-idiotype, par des méthodes physiques ou chimiques, dans ledit fluide biologique.

7. Procédé de préparation d'un kit pour le diagnostic in vitro du Lupus Erythémateux Disséminé, caractérisé en ce qu'il comprend l'association :
- d'au moins un peptide selon la revendication 1, ou un conjugué de ce peptide avec une molécule porteuse, ou encore un anticorps anti-idiotype selon la revendication 3;
- des réactifs pour la constitution d'un milieu propice à la réaction immunologique entre les peptides ou les anticorps anti-idiotypes et les autoanticorps éventuellement présents dans un échantillon biologique;
- d'un ou plusieurs réactifs éventuellement marqués aptes à réagir avec le ou les peptides ou les anticorps anti-idiotype pour la détection du complexe immunologique formé;
- le cas échéant, d'un milieu biologique de référence, tel qu'un sérum d'un patient d'un sujet en bonne santé.

## Claims (Claims for the following Contracting State(s): AT, BE, CH, DE, DK, FR, GB, GR, IT, LI, LU, NL, SE)

1. Peptide capable of reacting with antibodies against the polypeptide Sm-D, which are present in a biological sample of a patient suffering from systemic lupus erythematosus, this peptide being selected from (1) a peptide of the sequence:
XMKLVRFLMKLSHETVTIELKZ (I)
wherein X denotes a free NH₂ group and Z represents either a free OH group or a peptide group selected from the following groups having 1 to 5 amino acids: N, NG, NGT, NGTQ, NGTQV; and (2) a peptide of the sequence:
XKMTLKNREPVQLETLSIRGNRIRYZ (IV)
wherein X represents either a free NH₂ group or a peptide group selected from the following groups having 1 to 5 amino acids: V, AV, KAV, LKAV, KLHAV;
and Z represents either a free OH group or a peptide group selected from the following groups having 1 to 5 amino acids: F, FI, FIL, FILP, FILPD.

2. Antibodies directed against the Sm-D antigen, characterised in that they are recognised by the peptides according to claim 1.

3. Anti-idiotype antibodies formed against an antibody according to claim 2, characterised in that they specifically recognise the autoantibodies present in biological fluids, particularly sera, from patients suffering from systemic lupus erythematosus.

4. Antigenic composition containing at least one peptide according to claim 1, characterised in that it reacted immunologically with the autoantibodies present in biological fluids, notably sera from patients suffering from systemic lupus erythematosus.

5. Immunogenic composition containing at least one peptide according to claim 1 or a conjugate of this peptide with a carrier molecule, characterised in that it triggers the production of antibodies capable of recognising the Sm-D antigen.

6. Process for *in vivo* diagnosis of systemic lupus erythematosus in a biological fluid, characterised in that it comprises at least the following steps:
- contacting this biological fluid with at least one peptide according to claim 1 or a conjugate of this peptide with a carrier molecule or an anti-idiotype antibody according to claim 3, under conditions which allow the formation of an immunological complex;
- detecting the presence of an antigen-antibody immunological complex or an antibody-antibody anti-idiotype complex, by physical or chemical methods, in said biological fluid.

7. Kit for *in vitro* diagnosis of systemic lupus erythematosus, characterised in that it comprises:
- at least one peptide according to claim 1 or a conjugate of this peptide with a carrier molecule or an anti-idiotype antibody according to claim 3;
- reagents for producing a medium which is favourable to the immunological reaction between the peptide or anti-idiotype antibodies and the autoantibodies which may be present in a biological sample;
- one or more optionally labelled reagents capable of reacting with the or each peptide or the anti-idiotype antibodies in order to detect the immunological complex formed;
- if required, a reference biological medium such as a serum from a subject in good health.

## Claims (Claims for the following Contracting State(s): ES)

1. Process for preparing a peptide capable of reacting with antibodies against the polypeptide Sm-D, which are present in a biological sample of a patient suffering from systemic lupus erythematosus, this peptide being selected from (1) a peptide of the sequence:
XMKLVRFLMKLSHETVTIELKZ (I)
wherein X denotes a free NH₂ group and Z represents either a free OH group or a peptide group selected from the following groups having 1 to 5 amino acids: N, NG, NGT, NGTQ, NGTQV; and (2) a peptide of the sequence:
XKMTLKNREPVQLETLSIRGNRIRYZ (IV)
wherein X represents either a free NH₂ group or a peptide group selected from the following groups having 1 to 5 amino acids: V, AV, KAV, LKAV, KLKAV;
and Z represents either a free OH group or a peptide group selected from the following groups having 1 to 5 amino acids: F, FI, FIL, FILP, FILPD:
- either by solid phase peptide synthesis,
- or by condensation of the amino acid residues in the appropriate order,
- or by condensation of numerous fragments prepared beforehand, the reactive functions carried by the amino acid residues or the fragments, with the exception of the amino and carboxyl functions engaged in the peptide bond, being protected beforehand.

2. Process for preparing antibodies directed against the Sm-D antigen, recognised by the peptides according to claim 1, characterised in that they are obtained by means of a hybridoma or by immunising an animal against one of the said peptides defined in claim 1.

3. Process for preparing anti-idiotype antibodies formed against an antibody according to claim 2, characterised in that they specifically recognise the autoantibodies present in biological fluids, notably sera, from patients suffering from systemic lupus erythematosus.

4. Process for preparing an antigenic composition containing at least one peptide according to claim 1, characterised in that it reacted immunologically with the autoantibodies present in biological fluids, notably sera from patients suffering from systemic lupus erythematosus.

5. Process for preparing an immunogenic composition containing at least one peptide according to claim 1 or a conjugate of this peptide with a carrier molecule, characterised in that it triggers the production of antibodies capable of recognising the Sm-D antigen.

6. Process for *in vivo* diagnosis of systemic lupus erythematosus in a biological fluid, characterised in that it comprises at least the following steps:
- contacting this biological fluid with at least one peptide according to claim 1 or a conjugate of this peptide with a carrier molecule or an anti-idiotype antibody according to claim 3, under conditions which allow the formation of an immunological complex;
- detecting the presence of an antigen-antibody immunological complex or an antibody-antibody anti-idiotype complex, by physical or chemical methods, in said biological fluid.

7. Process for preparing a kit for *in vitro* diagnosis of systemic lupus erythematosus, characterised in that it comprises the combination of:
- at least one peptide according to claim 1 or a conjugate of this peptide with a carrier molecule or an anti-idiotype antibody according to claim 3;
- reagents for producing a medium which is favourable to the immunological reaction between the peptide or anti-idiotype antibodies and the autoantibodies which may be present in a biological sample;
- one or more optionally labelled reagents capable of reacting with the or each peptide or the anti-idiotype antibodies in order to detect the immunological complex formed;
- if required, a reference biological medium such as a serum from a subject in good health.

## Patentansprüche (Patentansprüche für folgende(n) Vertragsstaat(en): AT, BE, CH, DE, DK, FR, GB, GR, IT, LI, LU, NL, SE)

1. Peptid, welches mit den Antikörpern gegen das Polypeptid Sm-D zu reagieren vermag, welche in einer biologischen Probe eines an Lupus erythematodes disseminatus leidenden Subjekts vorhanden sind, welches Peptid ausgewählt ist aus
(1) einem Peptid der Sequenz:
XMKLVRFLMKLSHETVTIELKZ (I) in der X eine freie NH₂-Gruppe und Z entweder eine freie OH-Gruppe oder eine Peptidgruppe ausgewählt aus den folgenden Aminosäuregruppen 1 bis 5: N, NG, NGT, NGTQ, NGTQV bedeuten; und
(2) einem Peptid der Sequenz:
XKMTLKNREPVQLETLSIRGNRIRYZ (IV) in der X entweder eine freie NH₂-Gruppe oder eine Peptidgruppe ausgewählt aus den folgenden Aminosäuregruppen 1 bis 5: V, AV, KAV, LKAV, KLKAV, und Z entweder eine freie OH-Gruppe oder eine Peptidgruppe ausgewählt aus den folgenden Aminosäuregruppen 1 bis 5: F, FI, FIL, FILP, FILPD, bedeuten.

2. Gegen das Antigen Sm-D gerichtete Antikörper, **dadurch gekennzeichnet**, daß sie von den Peptiden nach Anspruch 1 erkannt werden.

3. Anti-idiotype Antikörper, die gegen einen Antikörper nach Anspruch 1 gerichtet sind, **dadurch gekennzeichnet**, daß sie in spezifischer Weise die Autoantikörper erkennen, die in biologischen Flüssigkeiten, insbesondere Seren von Patienten enthalten sind, die an Lupus erythematodes disseminatus erkrankt sind.

4. Antigene Zubereitung enthaltend mindestens ein Peptid nach Anspruch 1, **dadurch gekennzeichnet**, daß sie immunologisch mit den Antikörpern reagiert, die in biologischen Flüssigkeiten, insbesondere Seren von Patienten enthalten sind, die an Lupus erythematodes disseminatus erkrankt sind.

5. Immunogene Zubereitung, enthaltend mindestens ein Peptid nach Anspruch 1 oder ein Konjugat dieses Peptids mit einem Trägermolekül, **dadurch gekennzeichnet**, daß sie die Bildung von Antikörpern induziert, die das Antigen Sm-D erkennen können.

6. Verfahren zur in vivo-Diagnose von Lupus erythematodes disseminatus in einer biologischen Flüssigkeit, **dadurch gekennzeichnet**, daß es mindestens die folgenden Stufen umfaßt:
- das Inkontaktbringen dieser biologischen Flüssigkeit mit mindestens einem Peptid nach Anspruch 1 oder einem Konjugat dieses Peptids mit einem Trägermolekül, oder schließlich einem anti-idiotypen Antikörper nach Anspruch 3 unter Bedingungen, welche die Bildung eines immunologischen Komplexes ermöglichen;
- den Nachweis der Anwesenheit eines immunologischen Antigen-Antikörper-Komplexes oder eines Antikörper-anti-idiotyp-Antikörper-Komplexes in der biologischen Flüssigkeit mit Hilfe von physikalischen oder chemischen Methoden.

7. Reagenssatz für die in vitro-Diagnose von Lupus erythematodes disseminatus, **dadurch gekennzeichnet**, daß er folgendes umfaßt:
- mindestens ein Peptid nach Anspruch 1 oder ein Konjugat dieses Peptids mit einem Trägermolekül oder schließlich einen anti-idiotypen Antikörper nach Anspruch 3;
- Reagenzien zur Bildung eines für die immunologische Reaktion zwischen den Peptiden oder den anti-idiotypen Antikörpern und den eventuell in einer biologischen Probe vorhandenen Autoantikörpern günstigen Mediums;
- eines oder mehrere gegebenenfalls markierte Reagenzien, die dazu geeignet sind, mit dem oder den Peptiden oder den anti-idiotypen Antikörpern zu reagieren zum Nachweis des gebildeten immunologischen Komplexes;
- gegebenenfalls ein biologisches Vergleichsmedium, wie ein Serum eines Patienten eines gesunden Subjekts.

## Patentansprüche (Patentansprüche für folgende(n) Vertragsstaat(en): ES)

1. Verfahren zur Herstellung eines Peptids, welches mit den Antikörpern gegen das Polypeptids Sm-D zu reagieren vermag, die in einer biologischen Probe eines Subjekts enthalten ist, das an Lupus erythematodis disseminatus erkrankt ist, welches Peptid ausgewählt ist aus:
(1) einem Peptid der Sequenz:
XMKLVRFLMKLSHETVTIELKZ (I) in der X eine freie NH₂-Gruppe und Z entweder eine freie OH-Gruppe oder eine Peptidgruppe ausgewählt aus den folgenden Aminosäuregruppen 1 bis 5: N, NG, NGT, NGTQ, NGTQV bedeuten; und
(2) einem Peptid der Sequenz:
XKMTLKNREPVQLETLSIRGNRIRYZ (IV) in der X entweder eine freie NH₂-Gruppe oder eine Peptidgruppe ausgewählt aus den folgenden Aminosäuregruppen 1 bis 5: V, AV, KAV, LKAV, KLKAV, und Z entweder eine freie OH-Gruppe oder eine Peptidgruppe ausgewählt aus den folgenden Aminosäuregruppen 1 bis 5: F, FI, FIL, FILP, FILPD, bedeuten,
- entweder durch Peptidsynthese in fester Phase,
- oder durch Kondensation der Aminosäurereste in der geeigneten Reihenfolge,
- oder durch Kondensation mehrerer zuvor gebildeter Fragmente, wobei die reaktiven Funktionen, die von den Aminosäureresten oder den Fragmenten getragen werden, mit Ausnahme der an der Bildung der Peptidbindung beteiligten Amino- und Carboxylfunktionen, zuvor geschützt worden sind.

2. Verfahren zur Herstellung von gegen das Antigen Sm-D gerichteten Antikörpern, welche von den Peptiden nach Anspruch 1 erkannt werden, **dadurch gekennzeichnet**, daß sie mit Hilfe eines Hybridoms oder durch Immunisieren eines Tieres gegen eines der in Anspruch 1 definierten Peptide erhalten werden.

3. Verfahren zur Herstellung von anti-idiotypen Antikörpern, die gegen einen Antikörper nach Anspruch 2 gerichtet sind, **dadurch gekennzeichnet**, daß sie in spezifischer Weise die Autoantikörper erkennen, die in biologischen Flüssigkeiten, insbesondere Seren von an Lupus erythematodes disseminatus erkrankten Patienten enthalten sind.

4. Verfahren zur Herstellung einer antigenen Zubereitung, enthaltend mindestens ein Peptid nach Anspruch 1, **dadurch gekennzeichnet**, daß sie immunologisch mit den Antikörpern reagiert, die in biologischen Flüssigkeiten, insbesondere Seren von an Lupus erythematodes disseminatus erkrankten Patienten vorhanden sind.

5. Verfahren zur Herstellung einer immunogenen Zubereitung, enthaltend mindestens ein Peptid nach Anspruch 1, oder ein Konjugat dieses Peptids mit einem Trägermolekül, **dadurch gekennzeichne**t, daß sie die Produktion von Antikörpern induziert, welche das Antigen Sm-D erkennen können.

6. Verfahren zur in vivo-Diagnose von Lupus erythematodes disseminatus in einer biologischen Flüssigkeit, **dadurch gekennzeichnet**, daß es mindestens die folgenden Stufen umfaßt:
- das Inkontaktbringen dieser biologischen Flüssigkeit mit mindestens einem Peptid nach Anspruch 1 oder einem Konjugat dieses Peptids mit einem Trägermolekül, oder schließlich einem anti-idiotypen Antikörper nach Anspruch 3 unter Bedingungen, welche die Bildung eines immunologischen Komplexes ermöglichen;
- den Nachweis der Anwesenheit eines immunologischen Antigen-Antikörper-Komplexes oder eines Antikörper- anti-idiotyp -Antikörper-Komplexes in der biologischen Flüssigkeit mit Hilfe von physikalischen oder chemischen Methoden.

7. Verfahren zur Herstellung eines Reagenssatzes zur in vitro-Diagnose von Lupus erythematodes disseminatus, **dadurch gekennzeichnet**, daß er in Kombination folgendes umfaßt:
- mindestens ein Peptid nach Anspruch 1 oder ein Konjugat dieses Peptids mit einem Trägermolekül oder schließlich einen anti-idiotypen Antikörper nach Anspruch 3;
- Reagenzien zur Bildung eines für die immunologische Reaktion zwischen den Peptiden oder den anti-idiotypen Antikörpern und den eventuell in einer biologischen Probe vorhandenen Autoantikörpern günstigen Mediums;
- eines oder mehrere gegebenenfalls markierte Reagenzien, die dazu geeignet sind, mit dem oder den Peptiden oder den anti-idiotypen Antikörpern zu reagieren zum Nachweis des gebildeten immunologischen Komplexes;
- gegebenenfalls ein biologisches Vergleichsmedium, wie ein Serum eines Patienten eines gesunden Subjekts.
